(19) Europäisches Patentamt / European Patent Office / Office européen des brevets

(11) **EP 4 138 306 B1**

(12) **EUROPÄISCHE PATENTSCHRIFT**

(45) Veröffentlichungstag und Bekanntmachung des Hinweises auf die Patenterteilung:
**11.02.2026   Patentblatt 2026/07**

(21) Anmeldenummer: **21191668.9**

(22) Anmeldetag: **17.08.2021**

(51) Internationale Patentklassifikation (IPC):
**H04B 1/04** (2006.01)     **H04L 27/34** (2006.01)
**G01R 33/28** (2006.01)

(52) Gemeinsame Patentklassifikation (CPC):
**H04B 1/04; G01R 33/283; G01R 33/3621**

(54) **VERFAHREN ZUM ÜBERTRAGEN WENIGSTENS EINES SPRACHSIGNALS EINES PATIENTEN WÄHREND EINER MAGNETRESONANZ-BILDGEBUNGSUNTERSUCHUNG UND MAGNETRESONANZ-BILDGEBUNGSEINRICHTUNG**

METHOD FOR TRANSMITTING AT LEAST ONE VOICE SIGNAL OF A PATIENT DURING A MAGNETIC RESONANCE IMAGING EXAMINATION AND MAGNETIC RESONANCE IMAGING DEVICE

PROCÉDÉ DE TRANSMISSION D'AU MOINS UN SIGNAL VOCAL D'UN PATIENT PENDANT UN EXAMEN D'IMAGERIE PAR RÉSONANCE MAGNÉTIQUE ET DISPOSITIF D'IMAGERIE PAR RÉSONANCE MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.02.2023   Patentblatt 2023/08**

(73) Patentinhaber: **Siemens Healthineers AG**
**91301 Forchheim (DE)**

(72) Erfinder: **Bollenbeck, Jan**
**91330 Eggolsheim (DE)**

(74) Vertreter: **Siemens Healthineers**
**Patent Attorneys**
**Postfach 22 16 34**
**80506 München (DE)**

(56) Entgegenhaltungen:
**DE-A1- 102018 216 644     US-A- 3 508 155
US-A1- 2018 299 522**

- **ANONYMOUS: "Amplitude modulation - Wikipedia", 4 December 2019 (2019-12-04), XP055723381, Retrieved from the Internet <URL:https://en.wikipedia.org/w/index.php?title=Amplitude_modulation&oldid=929193037> [retrieved on 20200818]**

**Beschreibung**

Die Erfindung betrifft ein Verfahren zum Übertragen wenigstens eines Sprachsignals eines Patienten während einer Magnetresonanz-Bildgebungsuntersuchung, wobei das Sprachsignal von einer Sprachaufnahmeeinrichtung einer dem Patienten zugeordneten, drahtlosen Kommunikationseinrichtung aufgenommen und als ein Teil eines Kommunikationssignals an eine Empfangseinrichtung der Magnetresonanz-Bildgebungseinrichtung gesendet wird. Weiterhin betrifft die Erfindung eine Magnetresonanz-Bildgebungseinrichtung.

In einer Magnetresonanz-Bildgebungseinrichtung wird ein Patient für eine Untersuchung in der Regel auf einer Patientenliege angeordnet, welche sich innerhalb einer auch als Patientenaufnahme bezeichneten Durchgangsöffnung der Bildgebungseinrichtung befindet. Um eine Kommunikation des Patienten mit einem sich beispielsweise außerhalb eines Untersuchungsraums befindlichen Bediener der Bildgebungseinrichtung zu ermöglichen, ist es bekannt, über eine Kommunikationseinrichtung die Stimme des Patienten zu erfassen und an den Bediener zu übermitteln. Dabei kann z. B. ein Patientenmikrofon an Endbereichen eines Patientenaufnahmebereichs der Bildgebungseinrichtung angeordnet werden. Diese Mikrofone sind dabei jedoch in einem großen Abstand zum Patienten angeordnet, so dass vielfältige Störgeräusche, welche beispielsweise durch den Betrieb der Bildgebungseinrichtung erzeugt werden, ebenfalls erfasst und an den Bediener übertragen werden.

Aus DE 10 2018 216 644 A1 ist ein drahtloses Patientenmikrofon zur Verwendung in einer Magnetresonanz-Bildgebungseinrichtung bekannt. Dieses kann innerhalb einer Patientenaufnahme in unmittelbarer Nähe zu dem Mund des Patienten angeordnet werden, um eine bessere Sprachverständlichkeit zu erreichen. Zur Übertragung der aufgenommenen Sprache umfasst das Patientenmikrofon einen Modulator, mit welchem die aufgenommene Sprache zu einem analogen Kommunikationssignal moduliert wird. Zur Modulation des Kommunikationssignals wird dabei eine Amplitudenmodulation und/oder eine Winkelmodulation vorgeschlagen.

Magnetresonanz-Bildgebungseinrichtungen umfassen ein oder mehrere Empfangssysteme, um die für die Bildgebung notwendigen Signale zu empfangen. In der Regel sind diese Empfangssysteme und/oder die Auswertung der empfangenen Signale gegenüber elektromagnetischen Störungen sehr empfindlich. Zum Beispiel können aufgrund des Einsatzes von mehrdimensionalen Fourier-Analyse-Techniken bei der Auswertung der empfangenen Signale kontinuierliche Störsignale besonders hervortreten, wodurch es zu Artefakten in der Bildgebung kommen kann. Auch ein von einem drahtlosen Patientenmikrofon übertragenes Signal kann ein solches Störsignal darstellen und daher die Bildgebung durch die Magnetresonanz-Bildgebungseinrichtung negativ beeinflussen.

Aus US 2018 299 522 A1 ist eine Vorrichtung zur Rückgewinnung eines zeitlichen Bezugs in einer freilaufenden MRT-Empfangskette bekannt. Die Vorrichtung weist einen Zeitreferenzencoder und einen Zeitreferenzdecoder auf. Der Zeitreferenzencoder ist ausgelegt, ein Modulationssignal in Abhängigkeit von einem Referenztakt zu erzeugen, wobei das Modulationssignal für eine Korrelation mit einer zeitlichen Auflösung kleiner einer maximalen vorbestimmten Phasenabweichung und einem eindeutig zu identifizierendes Maximum ausgelegt ist. Der Zeitreferenzdecoder ist ausgelegt, über den ersten Signaleingang ein Empfangssignal in Abhängigkeit von dem Modulationssignal zu empfangen, eine Korrelation mit einem Referenzsignal auszuführen und ein Signal in Abhängigkeit von einem zeitlichen Bezug des Modulationssignals in dem Empfangssignal zu dem Referenzsignal zu generieren.

"Amplitude modulation - Wikipedia", 4. Dezember 2019 (2019-12-04) XP055723381, beschreibt die Amplitudenmodulation. Die Amplitudenmodulation ist eine Modulationstechnik, die in der elektronischen Kommunikation verwendet wird, am häufigsten zur Übertragung von Informationen über eine Funkträgerwelle. Bei der Amplitudenmodulation wird die Amplitude der Trägerwelle proportional zur Amplitude des übertragenen Nachrichtensignals variiert. Das Informationssignal ist beispielsweise eine Funktion des von einem Lautsprecher wiederzugebenden Tons oder der Lichtintensität von Pixeln eines Fernsehbildschirms.

Der Erfindung liegt daher die Aufgabe zugrunde, ein verbessertes Verfahren zum Übertragen wenigstens eines Sprachsignals eines Patienten während einer Magnetresonanz-Bildgebungsuntersuchung anzugeben, welches insbesondere einen störenden Einfluss des übertragenden Sprachsignals auf die Bildgebung reduziert.

Zur Lösung dieser Aufgabe ist bei einem Verfahren der eingangs genannten Art erfindungsgemäß vorgesehen, dass das Kommunikationssignal ein moduliertes Signal ist oder aus einem modulierten Signal erzeugt wird, wobei zur Erzeugung des modulierten Signals das Sprachsignal auf ein Trägersignal aufmoduliert wird, wobei das modulierte Signal durch eine Modulation mit Reduzierung des Pegels des Trägersignals erzeugt wird.

Eine Modulation mit Reduzierung des Pegels des Trägersignals kann auch als Modulation mit reduziertem Träger (engl. reduced carrier modulation) bezeichnet werden. Dabei wird der Pegel der Trägersignals auf einen festen Pegel, der größer als null ist, reduziert. Das Trägersignal kann dabei auf einen festen Pegel reduziert werden, welcher unterhalb des Trägerpegels für einen senderseitigen Modulator liegt. Der reduzierte Pegel des Trägersignals ist insbesondere kleiner als der Pegel eines Trägersignals, welches mit klassischer Amplitudenmodulation moduliert wird. Der reduzierte Trägerpegel wird als ein Teil der Kommunikationssignals an die Empfangseinrichtung übertragen und dient zum Beispiel als Referenzfrequenz. Der empfangene, pegelreduzierte Trägersignalanteil des Kommunikationssignals wird insbesondere in der Empfangseinrichtung für die Erzeugung eines rekonstruierten Trägersignals verwendet, wobei das rekonstruierte Trägersignal zur Demodulation des empfangenen und/oder gefilterten Kommunikationssignals benutzt wird.

Zur Erzeugung des Kommunikationssignals wird das Sprachsignal von einer Sprachaufnahmeeinrichtung der Kommunikationseinrichtung, insbesondere kontinuierlich, aufgenommen. Die Sprachaufnahmeeinrichtung kann zum Beispiel ein Mikrofon sein, welches Schall aus der unmittelbaren Umgebung des Patienten aufnimmt. Das von der Sprachaufnahmeeinrichtung erzeugte, elektrische Signal wird vorliegend als Sprachsignal bezeichnet, auch wenn nicht durchgehend Sprache aufgezeichnet wird, sondern nur in den Fällen, in denen der Patient spricht. Wenn der Patient nicht spricht, kann das Sprachsignal beispielsweise Umgebungsgeräusche und/oder Atemgeräusche des Pateinten beschreiben.

Das Sprachsignal wird in der Kommunikationseinrichtung als Modulationssignal auf das Trägersignal moduliert, wodurch ein moduliertes Signal erzeugt wird. Bei der Modulation wird der Pegel des Trägersignals gegenüber einer normalen Amplitudenmodulation mit vollständigem Trägerpegel reduziert, zum Beispiel um einen Faktor zwischen -10 dB und -40 dB, beispielsweise -30 dB. Das erzeugte modulierte Signal kann direkt als Kommunikationssignal verwendet werden. Alternativ kann das Kommunikationssignal aus dem modulierten Signal, zum Beispiel durch eine Filterung oder dergleichen, erzeugt werden. In beiden Fällen wird das Kommunikationssignal anschließend an die Empfangseinrichtung übermittelt. In der Empfangseinrichtung kann das Kommunikationssignal insbesondere mit Hilfe einer Rekonstruktion des Trägersignals demoduliert werden, so dass auch das rekonstruierte Sprachsignal in der Empfangseinrichtung digital und/oder analog zur Verfügung steht. Das Kommunikationssignal kann in der Empfangseinrichtung unmittelbar demoduliert werden oder es kann weiterverarbeitet, zum Beispiel gefiltert, werden. Das rekonstruierte Sprachsignal kann anschließend weiterverarbeitet, aufgezeichnet und/oder ausgegeben werden.

Die Verwendung einer Modulation mit Reduzierung des Pegels des Trägersignals hat den Vorteil, dass der durch das Trägersignal entstehende, kohärente Anteil des Kommunikationssignals reduziert werden kann. Bei einer konventionellen Zweiseitenband-Amplitudenmodulation mit vollständigem Träger (dual side band modulation with carrier, DSB-WC) verbleibt hingegen das Trägersignal unabhängig von einem Pegel des Modulationssignals im Signalspektrum, auch wenn zum Beispiel die Amplitude eines aufmodulierten Sprachsignals klein wird, beispielsweise wenn die Kommunikationseinrichtung nicht von dem Patienten besprochen wird.

Der Anteil des Trägersignals entspricht dabei einem kontinuierlichen, phasenkohärenten Kosinussignal, welches als ein kontinuierliches Störsignal die Bildgebung in der Magnetresonanz-Bildgebungseinrichtung beeinflussen kann. Eine Störung kann dabei beispielsweise dadurch entstehen, dass Harmonische des Trägersignalspektrums entweder unmittelbar in Empfangsbereiche der Bildgebungseinrichtung fallen, oder durch Intermodulation mit weiteren, zumindest zeitweise kontinuierlich verlaufenden Signalen in einem Empfangsbereich, einem Nebenempfangsbereich oder einem Alias-Band der Analog-DigitalWandlung erscheinen. Dies kann insbesondere auch dann auftreten, wenn die Frequenz des Trägersignals außerhalb des Frequenzspektrums für die Bildgebung liegt. Weiterhin können auch Verfahren zur Erzeugung einer Bewegungsinformation, beispielsweise ein Pilot-Ton-Verfahren oder Ähnliches, durch Harmonische des Trägersignals gestört werden.

Diese Störungen können entsprechend auch bei einer Frequenzmodulation auftreten. Auch bei der Frequenzmodulation verbleibt das Trägersignal unter Umständen, insbesondere wenn keine Modulation erfolgt, im Signalspektrum bestehen. Außerdem wird das Betragsspektrum einer Schmalband-Frequenzmodulation mit kleiner werdendem Modulationssignal dem Betragsspektrum einer Zweiseitenband-Amplitudenmodulation immer ähnlicher, so dass die bezüglich der Übermittlung mittels eines DSB-WC Signals geschilderte Problematik bei einer Frequenzmodulation im Wesentlichen analog besteht.

Die Erzeugung des von der Kommunikationseinrichtung gesendeten Kommunikationssignals durch eine Modulation mit Reduzierung des Pegels des Trägersignals verringert signifikant die Störungen bzw. die störenden Einflüsse auf die Bildgebung, welche durch die Übertragung des Kommunikationssignals von der Kommunikationseinrichtung an die Empfangseinrichtung der Bildgebungseinrichtung entstehen. Dieses Vorgehen eignet sich somit vorteilhaft für den Einsatz in einer Magnetresonanz-Bildgebungseinrichtung. Insbesondere kann dadurch die Kommunikationseinrichtung auch während Empfangsphasen der Magnetresonanz-Bildgebungseinrichtung betrieben werden, ohne die Bildgebung zu stören.

Ein zusätzlicher Vorteil des erfindungsgemäßen Verfahrens ist eine Verringerung der benötigten Sendeleistung, da verglichen mit einer konventionellen Amplitudenmodulation mit vollständigem Träger ein höherer Leistungsanteil in den die Information, also das Sprachsignal, umfassenden Seitenbändern des modulierten Signals bzw. des Kommunikationssignals liegt. Bei einer konventionellen Zweiseitenband-Amplitudenmodulation mit Träger befinden sich im besten Fall bei einem Modulationsindex $m = 1$ zwei Drittel der Leistung im unmodulierten Träger und nur ein Drittel der Leistung in den Seitenbändern. Mit kleiner werdendem Modulationsindex bleibt die Trägerleistung unverändert, während die Leistung auf den Seitenbändern weiterhin stetig abnimmt. Auch bei einer Frequenzmodulation wird die Amplitude des Trägers nicht verändert und die Gesamtleistung entspricht daher, unabhängig von der Modulation, stets der Leistung des unmodulierten Trägers.

Da während einer Bildgebungsuntersuchung der Patient in der Regel nicht permanent spricht, insbesondere um Bewegungen des Patienten und somit weitere störende Einflüsse auf die Bildgebung zu vermeiden, wird durch die Kommunikationseinrichtung in der Regel die meiste Zeit keine Sprache des Patienten aufgenommen. In einem solchen Fall werden als Sprachsignal in der Regel lediglich leise Störgeräusche empfangen. Obwohl das übertragene Kommunikationssignal in diesem Fall nur ein Sprachsignal bzw. ein Modulationssignal mit sehr geringer Amplitude umfasst, wird im Falle von Frequenzmodulation dieselbe bzw. im Fall einer konventionellen Amplitudenmodulation annähernd dieselbe Leistung in der Kommunikationseinrichtung umgesetzt, wie bei einem maximalen Pegel des Sprachsignals, welcher beispielsweise auftritt, wenn die Kommunikationseinrichtung bzw. ihre Sprachaufnahmeeinrichtung laut besprochen wird.

Durch die Verwendung einer Modulation mit Reduzierung des Pegels des Trägersignals für die Erzeugung des modulierten Signals kann daher auch der Leistungsanteil des Trägersignals reduziert werden, wodurch vorteilhaft die maximale Peak-Leistungsaufnahme bei der Erzeugung des Kommunikationssignals reduziert wird. Dies verringert entsprechend auch den Energieverbrauch in der Kommunikationseinrichtung und ermöglicht daher die Verwendung eines kleineren Energiespeichers, zum Beispiel eines kleineren Akkus, in der Kommunikationseinrichtung und somit eine kleinere Baugröße der Kommunikationseinrichtung. Alternativ kann auch eine längere Laufzeit der Kommunikationseinrichtung bei gleicher Energiespeichergröße erreicht werden.

Grundsätzlich wäre es zwar denkbar, auch eine Modulation mit vollständiger Unterdrückung des Pegels des Trägersignals einzusetzen. Diese weist jedoch den Nachteil auf, dass ohne Trägersignalanteil im Kommunikationssignal keine Synchronisation auf das Trägersignal in der Empfangseinrichtung möglich ist, so wie es bei der Verwendung eines Trägersignals mit reduziertem Pegel vorteilhaft erfolgen kann.

Bei einer Zweiseitenbandmodulation mit unterdrücktem Träger (double side band modulation supressed carrier, SSB-SC), ist der sendeseitige Schaltungsaufwand zwar geringer, jedoch kann es empfängerseitig zu Problemen beim Empfang des Sprachsignals führen, wenn die Phasenlage einer Frequenz eines Lokaloszillators von der Phasenlage des Trägersignals abweicht und mithin keine kohärente Demodulation möglich ist, wie sich durch die nachfolgende Betrachtung leicht erkennen lässt.

Ein DSB-SC Signal kann wie folgt beschrieben werden:

$$
\begin{aligned}
v_{DSB-SC}(t) &= K_{AM\_Mod} * \hat{V}_m * \cos(\omega_m t) * \hat{V}_c * \cos(\omega_c t) \\
&= K_{AM_{Mod}} * \frac{\hat{V}_m * \hat{V}_c}{2} * [\cos((\omega_c - \omega_m)t) + \cos((\omega_c + \omega_m)t)] \quad (1).
\end{aligned}
$$

Dabei bezeichnet $\hat{V}_m$ die Amplitude bzw. den Pegel des Modulationssignals, vorliegend des Sprachsignals, $\omega_m$ die Kreisfrequenz des Modulationssignals bzw. des Sprachsignals $\hat{V}_c$ die Amplitude bzw. den Pegel des Trägersignals und $\omega_c$ die Frequenz des Trägersignals. Die Konstante $K_{AM\_Mod}$ stellt ein Maß für die Modulator-Empfindlichkeit dar und besitzt die Einheit 1/V. Im Folgenden wird zur Vereinfachung der Darstellung die Konstante $K_{AM\_Mod}$ zu $\frac{1}{\hat{V}_c}$ gesetzt.

Bei Multiplikation des empfangenen Kommunikationssignals $v_{DSB-SC}(t)$ mit dem Signal eines Lokaloszillators $v_{LO}(t)$, welches sich als

$$
v_{LO}(t) = \hat{V}_{LO} * \cos(\omega_c t + \varphi) \quad (2)
$$

beschreiben lässt, wobei $\hat{V}_{LO}$ die Amplitude des Lokaloszillators beschreibt und $\varphi$ den Phasenversatz zwischen den Signalen $v_{LO}(t)$ und dem Trägersignal von $v_{DSB-SC}(t)$ beschreibt, ergibt sich

$$
\begin{aligned}
K_{AM\_Demod} &* v_{DSB-SC}(t) * v_{LO}(t) \\
&= K_{AM\_Demod} * \frac{\hat{V}_m * \hat{V}_{LO}}{4} * \left(\cos(\omega_m t + \varphi) + \cos((2\omega_c - \omega_m)t + \varphi)\right) \\
&+ K_{AM_{Demod}} * \frac{\hat{V}_m * \hat{V}_{LO}}{4} * \left(\cos(\omega_m t - \varphi) + \cos((2\omega_c + \omega_m)t + \varphi)\right) \quad (3),
\end{aligned}
$$

wobei sich nach einer Bandpassfilterung oder einer Tiefpassfilterung auf die Frequenz bzw. das Frequenzband des Modulationssignals für das empfangene Modulationssignal $v_{m,RX}(t)$

$$
v_{m,RX}(t) = \frac{\hat{V}_m}{2} * \cos(\omega_m t) * \cos(\varphi) \quad (4)
$$

ergibt.

Die Konstante $K_{AM\_Demod}$ stellt ein Maß für die Demodulator-Empfindlichkeit dar und besitzt die Einheit 1/V. Zur Vereinfachung der Darstellung wurde die Konstante $K_{AM\_Demod}$ zu $\frac{1}{\hat{V}_{LO}}$ gesetzt.

Wie in Formel (4) erkannt werden kann, ist die Amplitude des resultierenden Modulationssignals $v_{m,RX}(t)$ abhängig von der Phasenbeziehung $\varphi$ zwischen dem ursprünglichen Trägersignal und dem Lokaloszillatorsignal $v_{LO}(t)$. Diese Abhängigkeit entsteht, da die Phase des Trägersignals die Phasen der Beträge aus den beiden Seitenbändern gegenläufig beeinflusst, wie in Formel (3) zu sehen ist. Im Fall eines Phasenversatzes von $\varphi = \pm\pi/2$ kann es zu einer kompletten Auslöschung der Amplitude von $v_{m,RX}(t)$ kommen.

Auf eine umständliche Rekonstruktion des Trägersignals hinsichtlich Frequenz und Phase im Empfänger kann vorteilhaft verzichtet werden, indem die Modulation mit einer Reduzierung des Pegels des Trägersignals erfolgt. In diesem Fall wird ein Träger mit reduziertem Pegel übertragen, welcher einer Rekonstruktion des Trägersignals für die Demodulation des Kommunikationssignals im Empfänger ermöglicht.

Beispielsweise kann eine sogenannte Zweiseitenbandmodulation mit reduziertem Träger (dual side band modulation reduced carrier, DSB-RC) durchgeführt werden. Das in diesem Fall erhaltene Kommunikationssignal $v_{DSB-RC}(t)$ ergibt sich dabei wie folgt:

$$v_{DSB-RC}(t) = \left(k + \hat{V}_m * K_{AM\_Mod} * \cos(\omega_m t)\right) * \hat{V}_c * \cos(\omega_c t)$$
$$= \frac{\hat{V}_m}{2} * \cos((\omega_c - \omega_m)t) + k * \hat{V}_c * \cos(\omega_c t) + \frac{\hat{V}_m}{2} * \cos((\omega_c + \omega_m)t) \quad (5),$$

wobei k ein relatives Maß für die Trägeramplitude ist. Der Parameter k ist dabei größer 0 und kleiner 1.

Bei Verwendung eines Lokaloszillatorsignals $\hat{V}_{LO}(t)$, welches aus dem übertragenen Trägersignal mit dem reduzierten Pegel gewonnen werden kann, ergibt sich das demodulierte Signal

$$K_{AM\_Demod} * v_{DSB-RC}(t) * v_{LO}(t)$$
$$= K_{AM_{Demod}} * \frac{\hat{V}_m * \hat{V}_{LO}}{4} * \left(\cos\left(\omega_m t + \varphi\right) + \cos\left((2\omega_c - \omega_m)t + \varphi\right)\right)$$
$$+ \frac{k * K_{AM_{Demod}} * \hat{V}_c * \hat{V}_{LO}}{2} * \left(\cos(\varphi) + \cos(2\omega_c t + \varphi)\right)$$
$$+ K_{AM_{Demod}} * \frac{\hat{V}_m * \hat{V}_{LO}}{4} * \left(\cos\left(\omega_m t - \varphi\right) + \cos\left((2\omega_c + \omega_m)t + \varphi\right)\right) \quad (6),$$

welches entsprechend bei einer Bandpassfilterung auf die Frequenz bzw. den Frequenzbereich des Sprachsignals $\omega_m$ mit der Vereinfachung $K_{AM\_Demod} = \frac{1}{\hat{V}_{LO}}$ zu

$$v_{m,RX}(t) = \frac{\hat{V}_m}{2} * \cos(\omega_m t) * \cos(\varphi) \quad (7)$$

gefiltert wird.

Da der übermittelte, pegelreduzierte Restträger in der Empfangseinrichtung rekonstruiert werden kann, kann das Lokaloszillator-Signal vorteilhaft ohne Phasenversatz, d.h. mit $\varphi = 0$, rekonstruiert werden. Das Sprachsignal $v_{m,RX}(t)$ kann somit vorteilhaft in der Empfangseinrichtung auch ohne Amplitudenschwankungen rekonstruiert werden.

In einer Magnetresonanz-Bildgebungseinrichtung kann es passieren, dass das Kommunikationssignal nicht durchgehend empfangen werden kann, insbesondere, wenn die Magnetresonanz-Bildgebungsuntersuchung sich in der Anregungsphase einer Bildaufnahmesequenz befindet. In einer solchen Anregungsphase werden starke Hochfrequenz- und Gradientenmagnetfelder erzeugt, welche die Übermittlung des Kommunikationssignals aufgrund ihrer hohen Amplituden bzw. Feldstärken stören können. Die Verwendung einer Modulation mit Reduzierung des Pegels des Trägersignals für die Erzeugung des Kommunikationssignals ermöglicht ein kontinuierliches Übertragen des Kommunikationssignals, da keine oder im Wesentlichen keine Störungen in den Empfangsphasen erzeugt werden, so dass vorteilhaft kein Abschalten und Anschalten der Kommunikationseinrichtung bzw. einer Sendeeinrichtung der Kommunikationseinrichtung erfolgen muss. Dadurch kann der Schaltungsaufwand in der Kommunikationseinrichtung und der Magnetresonanz-Bildgebungseinrichtung vorteilhaft reduziert werden.

Die Verwendung einer Modulation mit Reduzierung des Pegels des Trägersignals für die Erzeugung des Kommunikationssignals ermöglicht zwar vorteilhaft eine schnelle und einfache empfängerseitige Synchronisation auf den Träger, um den Sprachempfang nach Abschalten der Gradientenfelder wieder zu ermöglichen. Bis zum Erzeugen des rekonstruierten Trägers kann die Sprachqualität aufgrund der von der Phasenbeziehung abhängenden Amplitude des demodulierten Sprachsignals jedoch unzureichend sein.

In der klinischen Anwendung können sich MR-Sequenzen typischerweise über mehrere Minuten, beispielsweise über 5 Minuten, erstrecken. Zumindest während der Sendephasen bzw. der Anregungsphasen der Magnetresonanz-Bildgebungseinrichtung kann das Kommunikationssignal, also auch das reduzierte Trägersignal, empfängerseitig nicht empfangen werden, da die Amplituden bzw. die Signalpegel der von der Magnetresonanz-Bildgebungseinrichtung gesendeten Sendepositionen zu groß sind. In einem solchen Fall geht die Synchronisation auch bei Verwendung eines reduzierten Trägers im Empfänger verloren. Während einer einzelnen Sendephase oder während einer mehrere Sendephase umfassenden MR-Sequenz kann somit der Empfang des Kommunikationssignals im Empfänger unterbrochen sein und/oder deaktiviert werden.

In einer bevorzugten Ausgestaltung der Erfindung kann vorgesehen sein, dass als Kommunikationssignal das reduzierte Trägersignal und ein Seitenband des modulierten Signals übertragen werden. Der Modulationsinhalt steckt jeweils vollständig in jedem der beiden Seitenbänder, so dass es grundsätzlich ausreicht, ausschließlich eines der beiden Seitenbänder zu übertragen.

Die Erzeugung des modulierten Signals kann als Einseitenbandmodulation mit reduziertem Träger (single side band modulation reduced carrier, SSB-RC) erfolgen. Dies weist den Vorteil einer weiteren Reduktion der benötigten Sendeleistung auf. Ein Nachteil der Verwendung einer Einseitenbandmodulation ist, dass sich eine aufwändigere senderseitige Schaltung ergibt. Insbesondere ist die senderseitige Signalaufbereitung sehr aufwändig, so dass in der drahtlosen Kommunikationseinrichtung aufwändige und vergleichsweise bauraumintensive, analoge Schaltungen benötigt werden würden.

Eine andere Möglichkeit ist eine senderseitige Erzeugung des modulierten Signals mittels DSB-RC, wobei eines der Seitenbänder des modulierten Signales zur Erzeugung des Kommunikationssignals gefiltert wird, so dass nur das reduzierte Trägersignal und eines der Seitenbänder übertragen wird. Dies hat den nachfolgend noch genauer erläuterten Vorteil, dass ein Signal mit einem Seitenband demoduliert werden kann, weist jedoch den Nachteil auf, dass sendeseitig unnötig Energie für die Erzeugung des anschließend gefilterten Seitenbands aufgebracht werden muss.

Als weitere Alternative kann vorgesehen sein, dass als Kommunikationssignal das reduzierte Trägersignal und zwei Seitenbänder des modulierten Signals übertragen werden, wobei im Empfänger vor einer Demodulation eines der Seitenbänder aus dem Kommunikationssignal gefiltert wird. Die Erzeugung des modulierten Signals kann also bevorzugt als Zweiseitenbandmodulation mit unterdrücktem Träger (DSB-RC) durchgeführt werden. Die Filterung eines der Seitenbänder des Kommunikationssignals kann empfängerseitig beispielsweise durch einen Bandpassfilter erfolgen.

Alle Varianten haben den Vorteil, dass die Demodulation nur unter Verwendung eines einzigen Seitenbands in der Empfangseinrichtung umgesetzt werden können. Wie in den Formeln (4) und (7) zu erkennen ist, umfassen bei der Übertragung von Zweiseitenbändern die jeweils senderseitig erhaltenen Signale des Sprachsignals $v_{m,RX}(t)$ jeweils einen Faktor $\cos(\varphi)$ und somit eine Abhängigkeit der Amplitude des demodulierten Sprachsignals von der Phasenbeziehung $\varphi$ zwischen dem senderseitig verwendeten Trägersignal und dem empfängerseitig erzeugten Signal eines Lokaloszillators.

Diese Abhängigkeit entsteht, da die Trägerphase die Phasen der Beträge aus den beiden Seitenbändern gegenläufig beeinflusst, wie in den Formeln (3) und (6) zu erkennen ist. Im Fall eines Phasenversatzes von $\varphi = \pm\,90°$ kommt es im schlimmsten Fall zu einer vollständigen Auslöschung des empfangenen Sprachsignals im Empfänger, so dass keine Übertragung vom Patienten zu einem Bediener möglich ist.

Die Demodulation unter Verwendung eines einzigen Seitenbands, welche wie vorangehend beschrieben wurde durch die Übertragung eines einzigen Seitenbandes oder durch die empfängerseitige Filterung eines von zwei Seitenbändern erfolgen kann, kann dem vorteilhaft entgegenwirken. Ein empfängerseitig vorliegendes Signal $v_{SSB-RC}(t)$, welches beispielsweise als Kommunikationssignal direkt übertragen wird oder aus der Bandpassfilterung eines Zweiseitenbandsignals $v_{DSB-RC}(t)$ erzeugt werden kann, lässt sich wie folgt darstellen:

$$v_{SSB-RC}(t) = k * \hat{V}_c * \cos(\omega_c t) + \frac{\hat{V}_m}{2}$$
$$* \cos((\omega_c$$
$$+ \omega_m)t) \quad (8). \text{Fehler! Verweisquelle konnte nicht gefunden werden.}$$

Wird dieses Signal demoduliert, das heißt mit dem Signal eines Lokaloszillators gemäß Formel (2) multipliziert, so ergibt sich

$$K_{AM\_Demod} * v_{SSB-RC}(t) * v_{LO}(t) = \frac{k * K_{AM\_Demod} * \hat{V}_c * \hat{V}_{LO}}{2} * (\cos(\varphi) + \cos(2\omega_c t + \varphi))$$
$$+ K_{AM\_Demod} * \frac{\hat{V}_m * \hat{V}_{LO}}{4} * \left(\cos(\omega_m t - \varphi) + \cos((2\omega_c + \omega_m)t + \varphi)\right) \quad (9).$$

Nach einer Bandpassfilterung auf die Frequenz des Sprachsignals $\omega_m$ bzw. das Frequenzband des Sprachsignals ergibt sich mit der Vereinfachung $K_{AM\_Demod} = \frac{1}{\hat{V}_{LO}}$ schließlich für das im Empfänger erhaltene Sprachsignal $v_{m,RX}(t)$

$$v_{m,RX}(t) = \frac{\hat{V}_m}{4} * \cos(\omega_m t - \varphi) \quad (10).$$

Vorteilhaft beeinflusst die Phasenlage nun nicht mehr die Amplitude, sondern nur noch die Phase des empfängerseitig erhaltenen Sprachsignals. Dies verbessert signifikant den Empfang des Sprachsignals, wenn keine korrekte Phasenbeziehung zwischen dem Signal des Lokaloszillators und dem senderseitig verwendeten Trägersignal vorliegt.

Während einer mehrminütigen MR-Sequenz kann die Frequenz eines Oszillators in der Kommunikationseinrichtung, welcher beispielsweise als temperaturkompensierter Kristalloszillator (temperature compensated crystal oscillator, TCXO) ausgeführt ist und zur Erzeugung des Trägersignals verwendet wird, leicht wandern und sich beispielsweise um wenige Hertz verschieben. Ein Frequenzversatz zwischen der Frequenz des internen Oszillators und der Frequenz des Lokaloszillators in der Empfangseinrichtung schlägt sich direkt in einem Frequenzversatz im resultierenden Sprachsignal $v_{m,RX}(t)$ nieder.

Aufgrund des vorangehend beschriebenen Demodulierens mit nur einem der Seitenbänder kann sich dabei jedoch nur die Tonhöhe des Sprachsignals ändern, wobei eine Änderung in der Größenordnung von wenigen Hertz bei der Übertragung von Sprachsignalen nicht störend wahrnehmbar ist. Ebenso ist eine sich zeitlich ändernde Phasenbeziehung bei der Übertragung von Sprachsignalen ohne Belang.

Um den Schaltungsaufwand in der Kommunikationseinrichtung gering zu halten, werden bevorzugt als Kommunikationssignal das reduzierte Trägersignal und zwei Seitenbänder des modulierten Signals übertragen, wobei im Empfänger vor einer Demodulation eines der Seitenbänder, also das obere Seitenband (upper side band, USB) oder das untere Seitenband (lower side band, LSB) herausgefiltert wird.

Erfindungsgemäß kann das modulierte Signal aus dem Trägersignal und dem Sprachsignal mit einem doppelt symmetrischen Mischer, insbesondere einer Gilbert-Zelle, erzeugt werden. Die Erzeugung eines modulierten Signals als DSB-RC-Signal kann vorteilhaft mit einem doppelt symmetrischen Mischer folgen, da die Pegelreduktion in einfach zu realisierender Weise durch einen DC-Offset am Eingang des Mischers eingestellt werden kann. Vorteilhaft kann dabei als doppelt symmetrischer Mischer eine Gilbert-Zelle eingesetzt werden, wodurch sich ein einfacher und robuster Aufbau der Modulationsschaltung der Kommunikationseinrichtung ergibt.

Erfindungsgemäß wird in der Empfangseinrichtung das reduzierte Trägersignal für eine Demodulation des Kommunikationssignals über eine Phasenregelschleife, insbesondere über eine in einem digitalen Signalprozessor realisierte Phasenregelschleife, rekonstruiert. Die Phasenregelschleife kann dabei einen numerisch-kontrollierten Oszillator (numerical controlled oscillator, NCO) als Lokaloszillator aufweisen.

Mittels der Phasenregelschleife ist es möglich, aus dem übertragenen, pegelreduzierten Trägersignal des Kommunikationssignals ein rekonstruiertes Trägersignal für die Demodulation des Kommunikationssignals zu erzeugen. Insbesondere kann dabei nach einem Einrasten der Phasenschleife eine Rekonstruktion des Trägersignals ohne Frequenzversatz und ohne Phasenversatz erfolgen, so dass vorteilhaft eine kohärente Demodulation möglich ist.

Erfindungsgemäß wird während einer Sendephase der Magnetresonanz-Bildgebungseinrichtung die Frequenz eines Oszillators der Phasenregelschleife auf einen Wert der Frequenz vor oder zu Beginn der Sendephase gehalten, wobei das rekonstruierte Trägersignal während der Sendephase für die Demodulation des Kommunikationssignals mit der gehaltenen Frequenz des Oszillators erzeugt wird. Dies ermöglicht, dass direkt nach Beendigung der Sendephase wieder ein Empfang des Sprachsignals vorliegt. Bis zum erneuten Einrasten der Phasenschleife kann ein geringer Frequenzversatz des empfängerseitig bestimmten Sprachsignals auftreten, welcher sich aber, wie vorangehend beschrieben wurde, nicht störend auf die Sprachverständlichkeit auswirkt, wenn die Demodulation nur mit einem Seitenband erfolgt.

Die Phasenregelschleife kann beispielsweise auf der Oszillatorfrequenz kurz vor Beginn oder zu Beginn der Sendephase gehalten werden, indem die Phasenregelschleife in einen Haltemodus, auch als Hold-Modus bezeichnet, gesetzt wird, wenn die Sendephase beginnt bzw. kurz bevor die Sendephase beginnt. Nach der Sendephase kann die Phasenregelschleife von dem Haltemodus zurück in einen Verfolgungsmodus, auch als Carrier-Track-Modus bezeichnet, geschaltet werden, so dass die Phasenregelschleife wieder einrasten kann und die Demodulation des Kommunikationssignals kohärent und somit ohne Frequenz- oder Phasenversatz erfolgen kann.

In einer bevorzugten Ausgestaltung der Erfindung kann vorgesehen sein, dass eine zum Empfang von während einer Bildgebungssequenz der Magnetresonanz-Bildgebungseinrichtung erzeugten Magnetresonanzsignalen ausgebildete Empfangseinrichtung zum Empfang des Kommunikationssignals verwendet wird. Dadurch ergibt sich insbesondere ein einfacher Aufbau einer Kommunikationsanordnung, da vorteilhaft für die Empfangsvorrichtung der Kommunikationsanordnung eine bereits in der Magnetresonanzeinrichtung vorhandene Einrichtung verwendet werden kann.

Die Frequenz des Trägersignals wird insbesondere derart gewählt, dass sie leicht außerhalb der während der Magnetresonanz-Bildgebung empfangenen Frequenzen liegt, jedoch noch durch die Empfangseinrichtung der Magnetresonanz-Bildgebungseinrichtung empfangen werden kann. Beispielsweise kann bei einer Bildgebungseinrichtung, welche aufgrund des von ihr erzeugten Magnetfeldes einen Empfangsbereich von 63,6 MHz $\pm$ 350 kHz aufweist, eine Trägerfrequenz zwischen 62,5 und 63 MHz gewählt werden. Auf diese kann entsprechend das Sprachsignal als Modulationssignal aufmoduliert werden, wobei die Bandbreite des Sprachsignals je nach gewünschter Qualität der Sprachsignalübertragung zum Beispiel zwischen 3 und 5 kHz liegt.

Vorteilhaft liegt das Sprachsignal nach Empfang in der Empfangseinrichtung als digitales Signal vor, so dass das Sprachsignal von den Bilddaten getrennt und, wie vorangehend beschrieben wurde, mit Hilfe eines oder mehrerer digitaler Signalprozessoren demoduliert werden kann. Insbesondere kann das Sprachsignal vor der Auswertung der Bilddaten ausgeleitet werden.

Für eine erfindungsgemäße Magnetresonanz-Bildgebungseinrichtung ist vorgesehen, dass sie eine Kommunikationsanordnung mit einer einem Patienten zuordenbaren, drahtlosen Kommunikationseinrichtung, wobei die Kommunikationseinrichtung eine Sprachaufnahmeeinrichtung zur Aufnahme eines Sprachsignals des Patienten aufweist, und einer Empfangseinrichtung zum Empfang eines von der Kommunikationseinrichtung aus dem Sprachsignal erzeugten Kommunikationssignals umfasst, wobei die Kommunikationsanordnung zur Durchführung eines erfindungsgemäßen Verfahrens ausgebildet ist.

Sämtliche vorangehend in Bezug zu dem erfindungsgemäßen Verfahren beschriebenen Vorteile und Ausgestaltungen gelten entsprechend für die erfindungsgemäße Magnetresonanz-Bildgebungseinrichtung und umgekehrt.

Weitere Vorteile und Einzelheiten der vorliegenden Erfindung ergeben sich aus den im Folgenden beschriebenen Ausführungsbeispielen sowie anhand der Zeichnungen. Dabei zeigen:

Fig. 1     ein Ausführungsbeispiel einer erfindungsgemäßen Magnetresonanz-Bildgebungseinrichtung,

Fig. 2     ein erstes Blockdiagramm zur Erläuterung eines Ausführungsbeispiels eines erfindungsgemäßen Verfahrens, und

Fig. 3     ein weiteres Blockdiagramm zur Erläuterung des Ausführungsbeispiels des erfindungsgemäßen Verfahrens.

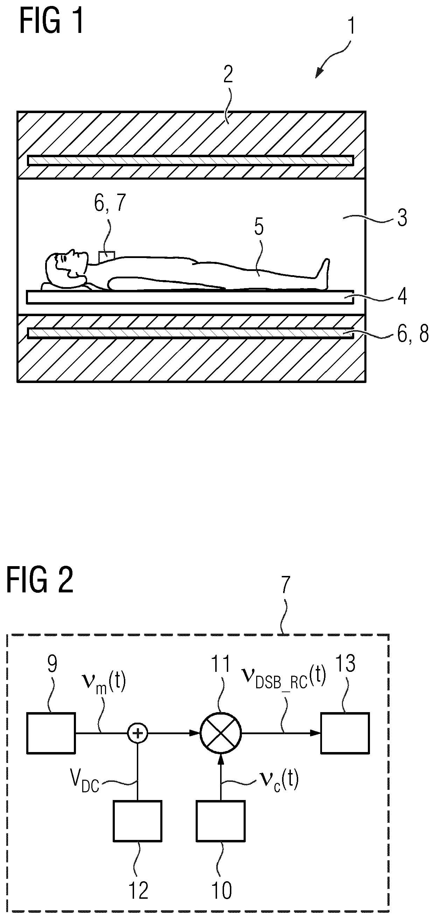

In Fig. 1 ist ein Ausführungsbeispiel einer erfindungsgemäßen Magnetresonanz-Bildgebungseinrichtung 1 dargestellt. Die Magnetresonanz-Bildgebungseinrichtung 1 umfasst eine Scanner-Einheit 2, in der ein Grundmagnet der Bildgebungseinrichtung 1 angeordnet ist. Weiterhin umfasst die Scanner-Einheit 2 eine Patientenaufnahme 3, in der eine Patientenlagerungsvorrichtung 4 mit einem Patienten 5 angeordnet werden kann.

Die Bildgebungseinrichtung 1 umfasst weiterhin eine Kommunikationsanordnung 6, welche eine dem Patienten 5 zuordenbare, drahtlose Kommunikationseinrichtung 7 und eine Empfangseinrichtung 8 aufweist. Die Empfangseinrichtung 8 ist dabei ebenfalls zum Empfang von während einer Bildgebungssequenz der Magnetresonanz-Bildgebungseinrichtung 1 erzeugten Magnetresonanzsignalen ausgebildet.

Die Empfangseinrichtung 8 wird weiterhin zum Empfang eines Kommunikationssignals verwendet, wobei das Kommunikationssignal von der Kommunikationseinrichtung 7 an die Empfangseinrichtung 8 übermittelt wird. Die Kommunikationseinrichtung 7 nimmt insbesondere kontinuierlich ein Sprachsignal des Patienten 5 bzw. ein Tonsignal aus der unmittelbaren Umgebung eines Kopfs des Patienten 5 auf und erzeugt aus dem Sprachsignal ein Kommunikationssignal. Das Kommunikationssignal wird von der Kommunikationseinrichtung 7 an die Empfangseinrichtung 8 übermittelt. Die Kommunikationseinrichtung 7 stellt bei der Übertragung des Kommunikationssignals bzw. des Sprachsignals folglich den Sender dar und die Empfangseinrichtung 8 den Empfänger.

In Fig. 2 ist ein schematisches Blockdiagramm der Kommunikationseinrichtung 7 dargestellt. In einem Ausführungsbeispiel eines Verfahrens zum Übertragen wenigstens eines Sprachsignals des Patienten 5 während einer Magnetresonanz-Bildgebungsuntersuchung durch die Magnetresonanz-Bildgebungseinrichtung 1 wird ein Kommunikationssignal aus dem Sprachsignal erzeugt und von der Kommunikationseinrichtung 7 an die Empfangseinrichtung 8 übermittelt. Das Kommunikationssignal wird in der Kommunikationseinrichtung 7 als ein moduliertes Signal oder aus einem modulierten Signal erzeugt.

Zur Erzeugung des modulierten Signals wird das Sprachsignal auf ein Trägersignal aufmoduliert, wobei das modulierte Signal durch eine Modulation mit Reduzierung des Pegels des Trägersignals erzeugt wird. Zur Aufnahme des Sprachsignals umfasst die Kommunikationseinrichtung eine als Mikrofon ausgebildete Spracherfassungseinrichtung 9, über die das Sprachsignal $v_m(t)$ aufgenommen werden kann. Das Sprachsignal $v_m(t)$ entspricht dabei dem aufgenommenen Schall in der unmittelbaren Umgebung des Patienten 5 und kann insbesondere vom Patienten 5 ausgegebene sprachliche Äußerungen umfassen.

Das Sprachsignal wird als ein Modulationssignal auf ein Trägersignal $v_c(t)$ aufmoduliert, wobei das Trägersignal durch einen Trägersignalgenerator 10 erzeugt wird. Der Trägersignalgenerator 10 kann beispielsweise ein temperaturkompensierter Kristalloszillator sein oder einen solchen umfassen.

Die Frequenz des Trägersignals $v_c(t)$ wird derart gewählt, dass sie leicht außerhalb der während der Magnetresonanz-Bildgebung empfangenen Frequenzen liegt, jedoch noch durch die Empfangseinrichtung 8 der Magnetresonanz-Bildgebungseinrichtung 1 empfangen werden kann. Beispielsweise kann bei einer Bildgebungseinrichtung 1, welche aufgrund des von ihr erzeugten Magnetfeldes einen Empfangsbereich von 63,6 MHz $\pm$ 350 kHz aufweist, eine Trägerfrequenz $\omega_c$ zwischen 62,5 MHz und 63 MHz gewählt werden. Auf diese kann entsprechend das Sprachsignal $v_m(t)$ als Modulationssignal aufmoduliert werden, wobei die Bandbreite des Sprachsignals je nach gewünschter Qualität der Sprachsignalübertragung zum Beispiel zwischen 3 kHz und 5 kHz liegt.

Im vorliegenden Ausführungsbeispiel wird das modulierte Signal mittels einer Zweiseitenbandmodulation mit reduziertem Träger (DSB-RC) erzeugt. Dazu umfasst die Kommunikationseinrichtung 7 einen doppelt symmetrischen Mischer 11, welcher das entsprechende modulierte Signal $v_{DSB-RC}(t)$ aus dem Sprachsignal $v_m(t)$ und dem Trägersignal $v_c(t)$ erzeugt. Der doppelt symmetrischer Mischer 11 kann insbesondere als eine Gilbert-Zelle ausgeführt sein.

Das Unterdrücken des Trägers erfolgt durch das Einbringen einer Asymmetrie in die Beschaltung des doppelt symmetrischen Mischers 11. Vorliegend wird ein DC-Offset $V_{DC}$ am Modulationssignaleingang, an dem das Sprachsignal $v_m(t)$ anliegt, angelegt. Der DC-Offset $V_{DC}$ wird dabei von einer Offset-Einrichtung 12, beispielsweise einer Spannungsquelle, der Kommunikationseinrichtung 7 erzeugt.

Das vom Mischer 11 erzeugte modulierte Signal $v_{DSB-RC}(t)$ wird dann über eine Sendevorrichtung 13 der Kommunikationseinrichtung 7, welche beispielsweise eine oder mehrere Antennen umfasst, an die Empfangseinrichtung 8 übermittelt. Bei der Modulation wird der Pegel des Trägersignals $v_c(t)$ verglichen zu einer Amplitudenmodulation mit vollständigem Trägersignal reduziert, zum Beispiel um einen Faktor zwischen -10 dB und -40 dB, beispielsweise um -30 dB. Dies ermöglicht es, auch während Empfangsphasen einer Magnetresonanz-Bildgebung ein Kommunikationssignal zu übertragen, ohne dass eine Beeinträchtigung der Bildgebung oder der Bildgebungsqualität auftritt.

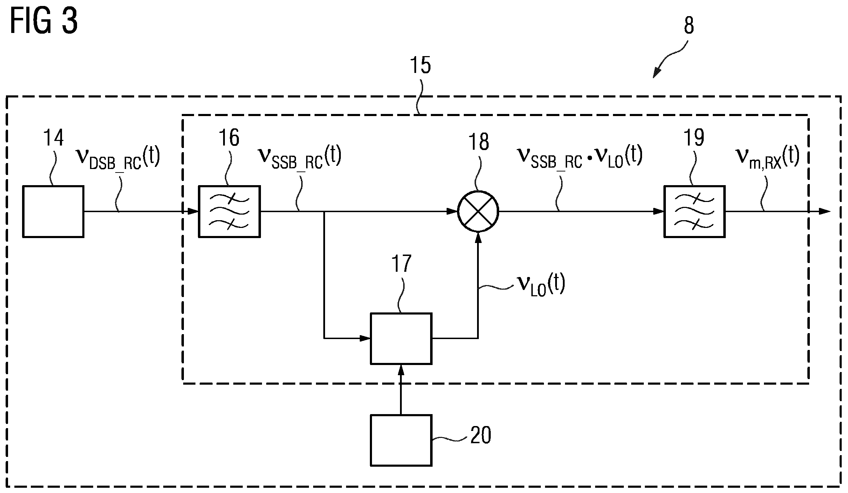

In Fig. 3 ist ein Blockdiagramm abgebildet, welches die empfängerseitige Signalverarbeitung in dem Ausführungsbeispiel des Verfahrens darstellt. Die Empfangseinrichtung 8 umfasst wenigstens eine Hochfrequenzantenneneinheit 14, welche ebenfalls zum Empfang der Bilddaten während einer Bildgebungssequenz der Bildgebungseinrichtung 1 verwendet werden kann. Weiterhin umfasst die Empfangseinrichtung 8 eine Recheneinheit 15, welche beispielsweise als digitaler Signalprozessor realisiert ist. Über die Hochfrequenzantenneneinheit 14 wird das Kommunikationssignal $v_{DSB-RC}(t)$ empfangen und einem ersten Bandpassfilter 16 zugeführt. Der Bandpassfilter 16 lässt aus dem empfangenen Zweiseitenbandsignal mit dem reduzierten Träger den Anteil des reduzierten Trägers sowie eines der Seitenbänder, beispielsweise das obere Seitenband, passieren und bildet somit ein Einseitenbandsignal mit reduziertem Träger $v_{SSB-RC}(t)$. Dieses enthält das Trägersignal $v_c(t)$ mit einer um den Faktor k reduzierten Amplitude bzw. eines um einen Faktor k reduzierten Pegel gemäß Formel (8).

Die Rekonstruktion des Trägersignals für die Demodulierung findet in einer Phasenregelschleife 17 statt, welche beispielsweise einen numerisch kontrollierten Oszillator (numerical controlled oscillator, NCO) als Lokaloszillator umfasst, dessen Frequenz an die Frequenz $\omega_c$ des Trägersignals angepasst wird. Weiterhin wird durch die Phasenregelschleife auch eine Anpassung der Phase vorgenommen, so dass im eingerasteten Zustand der Phasenregelschleife 17 kein Phasenversatz bei dem entsprechend erzeugten Lokaloszillatorsignal $v_{LO}(t)$ vorliegt.

Zur Demodulation des Sprachsignals $v_m(t)$ werden das bandpassgefilterte Kommunikationssignal $v_{SSB-RC}(t)$ sowie das Signal des Lokaloszillators $v_{LO}(t)$ in einem Mischer 18 multipliziert. Mit Hilfe eines zweiten Bandpassfilters 19 kann dann aus dem Produkt $v_{SSB-RC}(t) * v_{LO}(t)$ nach Formel (9) das Sprachsignal im Empfänger $v_{m,RX}(t)$ entsprechend Formel (10) herausgefiltert werden.

Da während der Anregungsphasen der Magnetresonanz-Bildgebungseinrichtung 1 kein Empfang des Kommunikationssignals und somit keine frequenz- oder phasenkorrekte Rekonstruktion des Trägersignals als Lokaloszillatorsignal $v_{LO}(t)$ in der Empfangseinrichtung 8 möglich ist, wird während einer Anregungsphase der Bildgebungseinrichtung 1 die Frequenz des internen Oszillators der Phasenregelschleife 17 auf einen Wert der Frequenz vor oder zu Beginn der Sendephase gesetzt. Das mit der festen Frequenz rekonstruierte Trägersignal $v_{LO}(t)$ kann somit auch kurzzeitig nach der Sendephase für die Demodulation des Kommunikationssignals, vorliegend des gefilterten Kommunikationssignals $v_{SSB-RC}(t)$, verwendet werden, so dass vorteilhaft auch bis zum erneuten Einrasten der Phasenregelschleife 17 auf einen übermittelten Trägersignalanteil des Kommunikationssignals bereits eine Sprachübermittlung erfolgen kann. Dies verbessert die Sprachübertragung insbesondere auch während den Bildgebungssequenzen Magnetresonanz-Bildgebungseinrichtung 1.

Für das Festsetzen der Frequenz des Lokaloszillators der Phasenregelschleife 17 kann die Phasenregelschleife 17 beispielsweise durch eine Steuerungseinrichtung 20 der Bildgebungseinrichtung 1 in einen Haltezustand versetzt werden, wenn eine, beispielsweise von der Steuerungseinrichtung 20 ausgelöste, MR-Sequenz bevorsteht oder beginnt. Nach Beendigung der Sequenz wird die Phasenregelschleife 17 beispielsweise wieder in einen Betriebsmodus, z. B. einen Carrier-Track-Modus, versetzt, so dass erneut ein Einrasten der Phasenschleife 17 auf das Trägersignal $v_C(t)$ bzw. den Trägersignalanteil des gefilterten Kommunikationssignals $v_{SSB-RC}(t)$ erfolgen kann.

In einem alternativen Ausführungsbeispiels des Verfahrens bzw. in einem alternativen Ausführungsbeispiel einer Bildgebungseinrichtung 1 kann vorgesehen sein, dass das Kommunikationssignal senderseitig bereits als mittels Einseitenbandmodulation mit reduziertem Träger (SSB-RC) gebildeten moduliertes Signal erzeugt und an die Empfangseinrichtung 8 übertragen wird. Alternativ ist eine Erzeugung eines DSB-RC-Signals, wie sie in Fig. 2 gezeigt wird, als moduliertes Signal möglich, wobei in der Kommunikationseinrichtung 7 mit Hilfe eines Bandpassfilters eines der Seitenbänder des modulierten Signals zur Erzeugung des anschließend übermittelten Kommunikationssignals ausgefiltert wird.

Wenn über die Hochfrequenzantenne 14 ein Einseitenbandmoduliertes Signal $v_{SSB-RC}(t)$ als Kommunikationssignal direkt empfangen wird, kann auf den ersten Bandpassfilter 16 verzichtet werden. Auch in einer solchen Ausgestaltung kann vorteilhaft erreicht werden, dass bei der Demodulation keine Phasenabhängigkeit der Amplitude des empfängerseitig ermittelten Sprachsignals $v_{m,RX}(t)$ auftritt, welche von dem Phasenwinkel zwischen dem Signal $v_{LO}(t)$ des Lokaloszillators und dem Trägersignal $v_m(t)$ abhängt, entsteht.

In einem weiteren, weniger bevorzugten Ausführungsbeispiel ist möglich, dass auf die Filterung eines der Seitenbänder durch den Bandpassfilter 16 verzichtet wird und das von der Hochfrequenzantenne 14 empfangene Zweiseitenbandsignal $v_{DSB-RC}(t)$ direkt dem Mischer 18 zugeführt wird. Auch in diesem Fall wird eine Übertragung des Kommunikationssignals, welches aus einem modulierten Signal durch eine Modulation mit unterdrücktem Träger erzeugt wurde, ermöglicht.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren zum Übertragen wenigstens eines Sprachsignals eines Patienten (5) während einer Magnetresonanz-Bildgebungsuntersuchung, wobei das Sprachsignal von einer Sprachaufnahmeeinrichtung (9) einer dem Patienten zugeordneten, drahtlosen Kommunikationseinrichtung (7) aufgenommen und zumindest als ein Teil eines Kommunikationssignals an eine Empfangseinrichtung (8) der Magnetresonanz-Bildgebungseinrichtung (1) gesendet wird, wobei das Kommunikationssignal ein moduliertes Signal ist oder aus einem modulierten Signal erzeugt wird, wobei zur Erzeugung des modulierten Signals das Sprachsignal auf ein Trägersignal aufmoduliert wird, dadurch gegenzeichnet , dass das modulierte Signal durch eine Modulation mit Reduzierung des Pegels des Trägersignals erzeugt wird, wobei in der Empfangseinrichtung (8) das reduzierte Trägersignal für eine Demodulation des Kommunikationssignals über eine Phasenregelschleife (17), rekonstruiert wird, wobei die Demodulation nur unter Verwendung eines einzigen Seitenbands in der Empfangseinrichtung erfolgt, wobei während einer Sendephase der Magnetresonanz-Bildgebungseinrichtung (1) die Frequenz eines Oszillators der Phasenregelschleife (17) auf einem Wert der Frequenz vor oder zu Beginn der Sendephase gehalten wird, wobei das rekonstruierte Trägersignal während der Sendephase für die Demodulation des Kommunikationssignals mit der gehaltenen Frequenz des Oszillators erzeugt wird.

2. Verfahren nach Anspruch 1, **dadurch gekennzeichnet, dass** als Kommunikationssignal das reduzierte Trägersignal und ein Seitenband des modulierten Signals übertragen werden.

3. Verfahren nach Anspruch 1, **dadurch gekennzeichnet, dass** als Kommunikationssignal das reduzierte Trägersignal und zwei Seitenbänder des modulierten Signals übertragen werden, wobei im Empfänger vor einer Demodulation eines der Seitenbänder aus dem Kommunikationssignal gefiltert wird.

4. Verfahren nach einem der vorangehenden Ansprüche, **dadurch gekennzeichnet, dass** das modulierte Signal aus dem Trägersignal und dem Sprachsignal mit einem doppelt symmetrischen Mischer (11), insbesondere einer Gilbert-Zelle, erzeugt wird.

5. Verfahren nach einem der vorangehenden Ansprüche, **dadurch gekennzeichnet, dass** in der Empfangseinrichtung (8) das reduzierte Trägersignal für eine Demodulation des Kommunikationssignals über eine in einem digitalen Signalprozessor realisierte Phasenregelschleife (17) rekonstruiert wird.

6. Verfahren nach einem der vorangehenden Ansprüche, **dadurch gekennzeichnet, dass** eine zum Empfang von während einer Bildgebungssequenz der Magnetresonanz-Bildgebungseinrichtung (1) erzeugten Magnetresonanzsignalen ausgebildete Empfangseinrichtung (8) zum Empfang des Kommunikationssignals verwendet wird.

7. Magnetresonanz-Bildgebungseinrichtung, umfassend eine Kommunikationsanordnung (6) mit einer einem Patienten (5) zuordenbaren, drahtlosen Kommunikationseinrichtung (7), wobei die Kommunikationseinrichtung (7) eine Sprachaufnahmeeinrichtung (9) zur Aufnahme eines Sprachsignals des Patienten (5) aufweist, und einer Empfangseinrichtung (8) zum Empfang eines von der Kommunikationseinrichtung aus dem Sprachsignal erzeugten Kommunikationssignals, wobei die Kommunikationsanordnung (6) zur Durchführung eines Verfahrens nach einem der vorangehenden Ansprüche ausgebildet ist.

## Claims

1. Method for transferring at least one speech signal of a patient (5) during a magnetic resonance imaging examination, wherein the speech signal is recorded by a speech recording facility (9) of a wireless communication facility (7) assigned to the patient and transmitted at least as part of a communication signal to a receive facility (8) of the magnetic resonance imaging facility (1), wherein the communication signal is a modulated signal or is generated from a modulated signal, wherein in order to generate the modulated signal the speech signal is modulated onto a carrier signal, **characterised in that** the modulated signal is generated by way of a modulation with reduction of the level of the carrier signal, wherein in the receive facility (8) the reduced carrier signal for a demodulation of the communication signal is reconstructed via a phase-locked loop (17), wherein the demodulation is only carried out using a single side band in the receive facility, wherein during a transmit phase of the magnetic resonance imaging facility (1), the frequency of an oscillator of the phase-locked loop (17) is held at a value of the frequency prior to or at the start of the transmit phase, wherein the reconstructed carrier signal is generated during the transmit phase for the demodulation of the communication signal with the held frequency of the oscillator.

2. Method according to claim 1, **characterised in that** the reduced carrier signal and a side band of the modulated signal are transferred as the communication signal.

3. Method according to claim 1, **characterised in that** the reduced carrier signal and two side bands of the modulated signal are transferred as the communication signal, wherein one of the side bands is filtered out of the communication signal in the receiver prior to a demodulation.

4. Method according to one of the preceding claims, **characterised in that** the modulated signal can be generated from the carrier signal and the speech signal with a double balanced mixer (11), in particular a Gilbert cell.

5. Method according to one of the preceding claims, **characterised in that** in the receive facility (8) the reduced carrier signal for a demodulation of the communication signal is reconstructed via a phase-locked loop (17) realised in a digital signal processor.

6. Method according to one of the preceding claims, **characterised in that** a receive facility (8) embodied to receive magnetic resonance signals generated during an imaging sequence of the magnetic resonance imaging facility (1) is used to receive the communication signal.

7. Magnetic resonance imaging facility, comprising a communication arrangement (6) with a wireless communication facility (7) which can be assigned to a patient (5), wherein the communication facility (7) has a speech recording facility (9) for recording a speech signal of the patient (5), and a receive facility (8) for receiving a communication signal generated by the communication facility from the speech signal, wherein the communication arrangement (6) is embodied to carry out a method according to one of the preceding claims.

## Revendications

1. Procédé de transmission d'au moins un signal vocal d'un patient (5) pendant un examen d'imagerie par résonnance magnétique, dans lequel le signal vocal est enregistré par un dispositif (9) d'enregistrement vocal d'un dispositif (7) de communication sans fil affecté au patient et est envoyé au moins comme partie d'un signal de communication à un dispositif (8) de réception du dispositif (1) d'imagerie par résonnance magnétique, dans lequel le signal de communication est un signal modulé ou est produit à partir d'un signal modulé, dans lequel, pour la production du signal modulé, le signal vocal est modulé sur un signal porteur, **caractérisé en ce que**

on produit le signal modulé par une modulation avec réduction du niveau du signal porteur, dans lequel, dans le dispositif (8) de réception, on reconstruit le signal porteur réduit pour une modulation du signal de communication par une boucle (17) à réglage de phase, dans lequel la démodulation n'a lieu qu'en utilisant une bande latérale unique du dispositif de réception,

dans lequel, pendant une phase d'émission du dispositif (1) d'imagerie par résonnance magnétique, on maintient la fréquence d'un oscillateur de la boucle (17) de réglage de phase à une valeur de la fréquence avant ou au début de la phase d'émission, dans lequel le signal porteur reconstruit est, pendant la phase d'émission, produit en maintenant la fréquence de l'oscillateur pour la démodulation du signal de communication.

2. Procédé suivant la revendication 1, **caractérisé en ce que** l'on transmet, comme signal de communication, le signal porteur réduit et une bande latérale du signal modulé.

3. Procédé suivant la revendication 1, **caractérisé en ce que** l'on transmet, comme signal de communication, le signal porteur réduit et deux bandes latérales du signal modulé, dans lequel, dans le récepteur, on filtre, avant une démodulation l'une des bandes latérales du signal de communication.

4. Procédé suivant l'une des revendications précédentes, **caractérisé en ce que** l'on produit le signal modulé à partir du signal porteur et du signal vocal par un mélangeur (11) symétrique doublé, en particulier par une cellule de Gilbert.

5. Procédé suivant l'une des revendications précédentes, **caractérisé en ce que**, dans le dispositif (8) de réception, on reconstruit le signal porteur réduit pour une démodulation du signal de communication par une boucle (17) à réglage de phase réalisée dans un processeur numérique de signal.

6. Procédé suivant l'une des revendications précédentes, **caractérisé en ce que** l'on utilise, pour la réception du signal de communication, un dispositif (8) de réception constitué pour la réception de signaux de résonnance magnétique produits pendant une séquence d'imagerie d'un dispositif (1) d'imagerie par résonnance magnétique.

7. Dispositif d'imagerie par résonnance magnétique, comprenant un agencement (6) de communication ayant un dispositif (7) de communication sans fil pouvant être associé à un patient (5), dans lequel le dispositif (7) de communication a un dispositif (9) d'enregistrement vocal pour l'enregistrement d'un signal vocal du patient (5), et un dispositif (8) de réception pour la réception d'un signal de communication produit par le dispositif de communication à partir du signal vocal, dans lequel l'agencement (6) de communication est constitué pour effectuer un procédé suivant l'une des revendications précédentes.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102018216644 A1
- US 2018299522 A1